# EUROPÄISCHE PATENTANMELDUNG

(11) **EP 3 750 981 A1**
(43) Veröffentlichungstag der Anmeldung: **16.12.2020**
(21) Anmeldenummer: 20185193.8
(22) Anmeldetag: 31.07.2015
(51) Int. Cl.: C12M 1/107, C12M 1/00, C12M 1/02, C12M 1/34

(54) **FLÜSSIGSUBSTRATBEHÄLTER FÜR EINE BIOGASANLAGE**

(30) Priorität: 04.08.2014 DE 102014011315
(62) Teilanmeldung aus: 15753897.6
(71) Anmelder: BTS Biogas Srl/GmbH, 39031 Bruneck (IT); Willeit, Jan, 39030 Enneberg (IT)
(72) Erfinder: Willeit, Jan, 39030 Enneberg (IT); Niederbacher, Michael, 39031 Bruneck (IT)
(74) Vertreter: Liebl, Thomas

(57) **Zusammenfassung**

Die Erfindung betrifft einen Flüssigsubstratbehälter für eine Biogasanlage, mit einem Behälterinnenraum, der mit einem Flüssigsubstrat befüllbar ist und mit einer den Behälterinnenraum bodenseitig begrenzenden, insbesondere wenigstens bereichsweise ebenen, Behälterbodenwand. Erfindungsgemäß ist vorgesehen, dass in der Behälterbodenwand wenigstens eine sich über einen vorgegebenen Teilbereich der Behälterbodenwand erstreckende, muldenartige Vertiefung ausgebildet ist, zu der und/oder in die wenigstens eine Abzugsleitung einer Abzugseinrichtung geführt ist. Die Abzugseinrichtung weist weiter eine Steuer- und/oder Regeleinrichtung auf, mittels der die Abzugseinrichtung dergestalt betätigbar ist, dass ein sich in der wenigstens einen muldenartigen Vertiefung betriebsmäßig ansammelndes Substrat-Sand-Gemisch über die wenigstens eine Abzugsleitung aus der wenigstens einen muldenartigen Vertiefung und damit aus dem Behälterinnenraum abziehbar ist, wobei die Abzugseinrichtung ferner wenigstens einen mit der wenigstens einen Abzugsleitung strömungsverbundenen Aufnahme- und/oder Absetzbehälter, insbesondere einen Separator, aufweist, in dem das über die wenigstens eine Abzugsleitung abgezogene Substrat-Sand-Gemisch aufnehmbar ist, insbesondere in eine Substratphase und in eine Sandphase trennbar ist.

## Beschreibung

Die Erfindung betrifft einen Flüssigsubstratbehälter für eine Biogasanlage nach dem Oberbegriff des Anspruchs 1.

Flüssigsubstratbehälter für Biogasanlagen sind allgemein bekannt. Diese weisen regelmäßig einen Behälterinnenraum auf, der mit einem Flüssigsubstrat befüllbar ist und eine den Behälterinnenraum bodenseitig begrenzende, insbesondere wenigstens bereichsweise ebene, Behälterbodenwand aufweist. An die Behälterbodenwand schließt sich eine den Behälterinnenraum umfangsseitig begrenzende Behälterseitenwand an, an die sich wiederum eine den nach oben offenen Behälter vorzugsweise gas- und flüssigkeitsdicht verschließende Abdeckung anschließt, die zum Beispiel durch ein Foliendach oder durch eine horizontale Abdeckwand, zum Beispiel eine Betondecke, gebildet sein kann.

Ein derartig aufgebauter Flüssigsubstratbehälter kann zum Beispiel ein herkömmlicher Fermenterbehälter sein, in dem das zu vergärende Flüssigsubstrat aufgenommen ist. Ebenso kann ein derartiger Flüssigsubstratbehälter ein Endlagerbehälter sein, in dem das bereits vergärte Flüssigsubstrat aufgenommen ist. In derartigen Flüssigsubstratbehältern ist regelmäßig wenigstens ein Rührwerk als Strömungserzeugungseinrichtung zugeordnet, mittels denen das im Flüssigsubstratbehälter aufgenommene Flüssigsubstrat zu dessen Durchmischung in eine gezielte Strömungsbewegung versetzt werden kann.

Insbesondere in Verbindung mit stark feststoffbelasteten Flüssigsubstraten besteht jedoch die Gefahr, dass sich diese Feststoffe als Substrat-Sand-Gemisch am Boden des Flüssigsubstratbehälters absetzen und sich dort ansammeln. Das sich behälterbodenseitig ansammelnde Substrat-Sand-Gemisch, wobei die Begrifflichkeit "Sand" hier ausdrücklich in einem weiten umfassenden Sinne zu verstehen ist, beeinträchtigt den praktischen Betrieb eines derartigen Flüssigsubstratbehälters in einer Biogasanlage erheblich, so dass das sich betriebsmäßig ansammelnde Substrat-Sand-Gemisch regelmäßig entfernt werden muss. Hierzu ist es bereits allgemein bekannt, einen derartigen Flüssigsubstratbehälter außer Betrieb zu setzen und den Behälter zu entleeren, um das bodenseitig angesammelte Substrat-Sand-Gemisch zu entfernen. Insbesondere bei längeren Betriebszeiten besteht dabei die Gefahr, dass das Substrat-Sand-Gemisch derart an die Bodenwand anbackt, dass das Entfernen des Substrat-Sand-Gemisches nur mit erheblichem Aufwand möglich ist. Ersichtlich handelt es sich hierbei um eine aufwendige und teure Prozedur, wobei zudem der Flüssigsubstratbehälter für eine bestimmte Zeit nicht im Betrieb der Biogasanlage zur Verfügung steht.

Demgegenüber ist es Aufgabe der vorliegenden Erfindung, einen Flüssigsubstratbehälter für eine Biogasanlage zu schaffen, mittels dem das sich im Substratbehälter betriebsmäßig ansammelnde Substrat-Sand-Gemisch auf einfache, funktionssichere und effektive Weise entfernbar ist.

Diese Aufgabe wird gelöst durch die Merkmale des Patentanspruchs 1. Vorteilhafte Ausgestaltungen sind Gegenstand der Unteransprüche.

Gemäß Anspruch 1 wird ein Flüssigsubstratbehälter für eine Biogasanlage vorgeschlagen, der einen Behälterinnenraum aufweist, der mit einem Flüssigsubstrat befüllbar ist und eine den Behälterinnenraum bodenseitig begrenzende, insbesondere wenigstens bereichsweise ebene, Behälterbodenwand aufweist. Erfindungsgemäß ist vorgesehen, dass in der Behälterbodenwand wenigstens eine sich über einen vorgegebenen Teilbereich der Behälterbodenwand erstreckende, muldenartige Vertiefung ausgebildet ist, zu der und/oder in die wenigstens eine Abzugsleitung einer Abzugseinrichtung geführt ist. Die Abzugseinrichtung weist eine Steuer- und/oder Regeleinrichtung auf, mittels der die Abzugseinrichtung dergestalt betätigbar ist, dass ein sich in der wenigstens einen muldenartigen Vertiefung betriebsmäßig ansammelndes Substrat-Sand-Gemisch über die wenigstens eine Abzugsleitung aus der wenigstens einen muldenartigen Vertiefung und damit aus dem Behälterinnenraum abziehbar ist. Die Abzugseinrichtung weist ferner wenigstens einen mit der wenigstens einen Abzugsleitung strömungsverbundenen Aufnahme- und/oder Absetzbehälter, insbesondere einen Separator, auf, in dem das über die wenigstens eine Abzugsleitung abgezogene Substrat-Sand-Gemisch aufnehmbar ist, insbesondere in Verbindung mit einem Separator in eine Substratphase und in eine Sandphase trennbar ist.

Die wenigstens eine bodenseitige muldenartige Vertiefung ermöglicht eine gezielte Ansammlung des betriebsmäßig anfallenden Substrat-Sand-Gemisches, weil die im Flüssigsubstrat enthaltenen Schweb- und Feststoffe, die auf den Boden absinken, dazu tendieren, sich an der tiefsten Stelle der Behälterbodenbodenwand anzureichern. Die gezielte Ansammlung des unerwünschten Substrat-Sand-Gemisches in der wenigstens einen muldenartigen Vertiefung ermöglicht des Weiteren ein gezieltes, insbesondere automatisiertes Abziehen dieser unerwünschten Ansammlungen aus der wenigstens einen muldenartigen Vertiefung durch Betätigung der Abzugseinrichtung mittels der Steuer- und/oder Regeleinrichtung. Die Betätigung der Abzugseinrichtung mittels der Steuer- und/oder Regeleinrichtung kann zu definierten Zeiten und/oder für eine definierte Zeitdauer erfolgen. Der Abzug des sich in der wenigstens einen muldenartigen Vertiefung betriebsmäßig ansammelnden Substrat-Sand-Gemisches über die wenigstens eine Abzugsleitung kann dabei besonders vorteilhaft während des laufenden Betriebes eines Flüssigsubstratbehälters erfolgen, das heißt, dass der Flüssigsubstratbehälter nicht außer Betrieb gesetzt und entleert werden muss, um das sich dort ansammelnde, unerwünschte Substrat-Sand-Gemisch abzuziehen.

Der Aufnahme- und/oder Absetzbehälter ist vorzugsweise ein Separator, in dem das über die wenigstens eine Abzugsleitung abgezogene Substrat-Sand-Gemisch in eine Substratphase und in eine Sandphase auftrennbar ist. Damit kann auf einfache Weise sichergestellt werden, dass das in Verbindung mit der Sandphase abgezogene Substrat wiedergewonnen werden kann und zum Beispiel über wenigstens eine Rückführleitung vom Separator zum Flüssigsubstratbehälter zurückgefördert werden kann. Alternativ oder zusätzlich kann das so gewonnene Substrat aber auch in jeden anderen geeigneten bzw. vergleichbaren Flüssigsubstratbehälter gefördert werden. Die unerwünschte Sandphase wird dagegen vom Separator abgezogen und zum Beispiel einer weiteren Verwendung zugeführt bzw. entsorgt. An dieser Stelle sei nochmals erwähnt, dass die Begrifflichkeit "Sand" hier ausdrücklich in einem umfassenden Sinne zu verstehen ist und jedwede Schweb- bzw. Feststoffe umfassen soll, die sich sedimentartig absetzen und bodenwandseitig in einem Flüssigsubstratbehälter ansammeln und anreichern können.

Sowohl die Abzugsleitung als auch die Absaugleitung können grundsätzlich auf unterschiedlichste Weise ausgebildet werden, wobei die Ausbildung der wenigstens einen Abzugsleitung durch eine Absaugleitung einer Absaugeinrichtung, zum Beispiel einer Pumpeinrichtung mit Pumpe, bevorzugt ist. Gemäß einer besonders bevorzugten konkreten Ausgestaltung ist die Absaugeinrichtung eine Vakuum- und/oder Unterdruck-Absaugeinrichtung, die eine Vakuum- und/oder Unterdruckpumpe und/oder die einen Vakuum- und/oder Unterdruckbehälter als Separator aufweist, in dem das Substrat-Sand-Gemisch mit einem gegenüber dem atmosphärischen Druck geringeren Druck aufgenommen ist. Mittels einer derartigen Vakuum- und/oder Unterdruck-Absaugeinrichtung kann das pumpbare Substrat-Sand-Gemisch auf einfache und funktionssichere Weise über die wenigstens eine Absaugleitung aus der wenigstens einen muldenartigen Vertiefung abgesaugt werden.

Dem Flüssigsubstratbehälter kann wenigstens eine Strömungserzeugungseinrichtung, zum Beispiel wenigstens ein Rührwerk zugeordnet sein bzw. der Flüssigsubstratbehälter kann wenigstens eine derartige Strömungserzeugungseinrichtung aufweisen. Mit dieser wenigstens einen Strömungserzeugungseinrichtung kann ein im Flüssigsubstratbehälter aufgenommenes Flüssigsubstrat mit einer definiert vorgegebenen Substratströmungsrichtung und/oder einer definiert vorgegebenen Substratströmungsgeschwindigkeit oberhalb der und/oder entlang der Behälterbodenwand strömen. Dadurch kann dem im Flüssigsubstratbehälter aufgenommenen Flüssigsubstrat eine solche gezielte Strömung aufgeprägt werden, die das Absetzen und Ansammeln der Feststoffe als Substrat-Sand-Gemisch in der wenigstens einen muldenartigen Vertiefung begünstigt. Gemäß einer besonders bevorzugten ersten konkreten Ausgestaltung kann dabei vorgesehen sein, dass die wenigstens eine Strömungserzeugungseinrichtung so im Flüssigsubstratbehälter angeordnet ist, dass das Flüssigsubstrat bei deren Betätigkeit in eine, vorzugsweise um eine senkrechte Behälterachse, rotierende Strömung versetzt wird.

Die wenigstens eine muldenartige Vertiefung kann ebenfalls grundsätzlich auf unterschiedlichste Art und Weise ausgebildet sein, zum Beispiel gekrümmt oder spiralförmig in der Behälterbodenwand verlaufen. Besonders bevorzugt ist jedoch eine Ausgestaltung, bei der wenigstens eine muldenartige Vertiefung durch wenigstens eine in der Behälterbodenwand ausgebildete Abzugsrinne, bevorzugt durch eine längliche bzw. langgestreckte Abzugsrinne, höchst bevorzugt durch eine im Wesentlichen geradlinig oder wenigstens in einem Teilbereich gekrümmt verlaufende langgestreckte Abzugsrinne, gebildet ist. Die Begrifflichkeit langgestreckt meint dabei jede geradlinige bzw. gegebenenfalls auch gekrümmte Erstreckung einer Abzugsrinne, bei der die Längsseiten länger sind als die Breit- bzw. Schmalseiten der Abzugsrinne. Eine derartige Abzugsrinne kann auf einfache Weise in einer Behälterbodenwand ausgebildet werden und begünstigt ein Absetzen der Feststoffe als Substrat-Sand-Gemisch in der Behälterbodenwand.

Die wenigstens eine Abzugsrinne ist bevorzugt so in der Behälterbodenwand angeordnet, dass diese im Strömungsweg des über die Behälterbodenwand strömenden Flüssigsubstrates liegt. Besonders bevorzugt ist dabei vorgesehen, dass mehrere, in Substratströmungsrichtung voneinander beabstandete Abzugsrinnen so im Behälterinnenraum angeordnet sind, dass diese im Strömungsweg des über die Behälterbodenwand strömenden Flüssigsubstrates liegen. Damit wird eine effektive Abscheidung der Feststoffe im Flüssigsubstrat in der wenigstens einen Abzugsrinne möglich. Besonders vorteilhaft ist dabei eine Ausgestaltung, bei der die wenigstens eine langgestreckte Abzugsrinne bezüglich ihrer gegenüber der Schmalseite längeren Längsseite im Wesentlichen quer zur Substratströmungsrichtung im Behälterinnenraum ausgerichtet und angeordnet ist. Auch hier ist wieder eine Ausführungsform besonders vorteilhaft, bei der nicht nur eine, sondern mehrere in Substratströmungsrichtung voneinander beabstandete langgestreckte Abzugsrinnen bezüglich ihrer Längsseite im Wesentlichen quer zur Substratströmungsrichtung im Behälterinnenraum angeordnet sind. Gegebenenfalls kann den Abzugsrinnen auch ein erhaben von der Behälterbodenwand abragendes Ablenkelement zugeordnet sein, das das Ansammeln und Absetzen der Feststoffe in der zugeordneten Abzugsrinne begünstigt. Ein derartiges Ablenkelement kann zum Beispiel entlang der Längsseite einer langgestreckten Abzugsrinne angeordnet sein und zum Beispiel schräg zur Abzugsrinne hin abfallen. Ein derartiges Ablenkelement kann sich lediglich über einen Teilbereich der Längsseite der Abzugsrinne erstrecken oder aber auch lediglich abschnittsweise angeordnet sein oder sich aber auch über die gesamte Länge einer Abzugsrinne erstrecken. Ebenso können zu beiden Seiten einer Abzugsrinne derartige Ablenkelemente angeordnet sein, ebenso wie ein derartiges Ablenkelement aber auch lediglich auf einer Seite der Abzugsrinne angeordnet sein kann, und hier dann bevorzugt auf der in Strömungsrichtung zweiten Längsseite der Abzugsrinne.

Wie bereits zuvor ausgeführt, gibt es grundsätzlich verschiedenste Möglichkeiten eine oder mehrere Abzugsrinnen behälterbodenwandseitig anzuordnen. Eine besonders effektive Lösung sieht vor, dass eine einzige Abzugsrinne oder wenigstens zwei voneinander beabstandete in Längsrichtung hintereinanderliegende Abzugsrinnen vorgesehen ist oder sind, die sich als diametral verlaufende Abzugsrinne zwischen diametral gegenüberliegenden Bodenwandbereichen des Flüssigsubstratbehälters erstreckt. Mit einer derartigen diametral verlaufenden Abzugsrinne ist sichergestellt, dass sich diese im Wesentlichen über den gesamten Durchmesser eines Flüssigsubstratbehälters erstreckt und somit eine insgesamt effiziente Ansammlung und Anreicherung der Feststoffe als Substrat-Sand-Gemisch in der wenigstens einen Abzugsrinne sichergestellt ist. Besonders vorteilhaft ist ein derartiger Aufbau in Verbindung mit einem Flüssigsubstratbehälter, insbesondere einem eine zylindrische Innenkontur aufweisenden Flüssigsubstratbehälter, der eine senkrechte Behälterachse, bevorzugt eine senkrechte Behältermittelachse, aufweist, um die das Flüssigsubstrat bei einer Betätigung der wenigstens einen Strömungserzeugungseinrichtung rotiert, wobei sich die, durch die wenigstens eine Abzugsrinne ausgebildete diametral verlaufende Abzugsrinne dann hier zu beiden Seiten der senkrechten Behälterachse erstreckt. Dies bewirkt, dass ein um senkrechte Behälterachse rotierendes Substratteil des Flüssigsubstrates die diametral verlaufende Abzugsrinne während eines 360°-Umlaufs um die senkrechte Behälterachse zweimal, zum Beispiel bei 0° und 180°, überstreicht, was eine besonders effektive und sichere Abscheidung der Feststoffe in der wenigstens einen Abzugsrinne sicherstellt.

Ein derartiger, eine diametral verlaufende Abzugsrinne aufweisender Flüssigsubstratbehälter ermöglicht somit eine effektive Feststoffabscheidung, was auch mit einem Flüssigsubstratbehälter, insbesondere einem, eine zylindrische Innenkontur aufweisenden Flüssigsubstratbehälter, möglich ist, der eine senkrechte Behälterachse, insbesondere eine senkrechte Behältermittelachse, aufweist, um die das Flüssigsubstrat bei einer Betätigung der wenigstens einen Strömungserzeugungseinrichtung rotiert, und bei dem mehrere voneinander in Strömungsrichtung beabstandete Abzugsrinnen vorgesehen sind, die sich von der senkrechten Behälterachse weg radial bzw. sternförmig nach außen erstrecken. Die umfangsseitige Beabstandung der Abzugsrinne ist dabei bevorzugt jeweils gleich, kann gegebenenfalls aber auch unterschiedlich sein. Auch mit einem derartigen Aufbau, der vor allem auch eine Ergänzung der diametral verlaufenden Abzugsrinne bedeuten kann, wird eine besonders effektive und gezielte Abscheidung von Feststoffen möglich. Ein derartiger Aufbau mit mehreren Abzugsrinnen eignet sich insbesondere für relativ großvolumige Behälter.

Die wenigstens eine Abzugsrinne selbst kann grundsätzlich jede geeignete Geometrie aufweisen. Besonders bevorzugt weist die wenigstens eine Abzugsrinne einen V-förmigen oder trichterförmigen Querschnitt auf, der den Abzug bzw. die Absaugung des Substrat-Sand-Gemisches aus der Abzugsrinne begünstigt.

Beispielsweise kann die wenigstens eine Abzugsleitung durch wenigstens eine, bezogen auf die Behälterhochachse, in einer Horizontalebene auf Höhe der wenigstens einen Abzugsrinne verlaufende horizontale Abzugsleitung gebildet sein, die seitlich, insbesondere in einem Abzugsrinnen-Seitenwandbereich, in die zugeordnete Abzugsrinne einmündet. Mit einer derartigen seitlichen Einmündung ist die Gefahr eines Verstopfens der Abzugsleitung deutlich reduziert, so dass hier auch Abzugsleitungen mit einem relativ kleinen Querschnitt verwendet werden können. Letzteres ist insbesondere dann von Vorteil, wenn in Verbindung mit den langgestreckten Abzugsrinnen gemäß einer besonders bevorzugten Ausgestaltung mehrere voneinander in Abzugsrinnenlängsrichtung beabstandete Abzugsleitungen in die wenigstens eine Abzugsrinne einmünden. Derartige mehrere beabstandete, in eine Abzugsrinne einmündende Abzugsleitungen bewirken einen besonders funktionssicheren, automatisierbaren Abzug des Substrat-Sand-Gemisches aus der jeweiligen Abzugsrinne. Beispielsweise kann gemäß einer konkreten Ausgestaltung jeweils eine Abzugsleitung in die in Abzugsrinnenlängsrichtung gesehen gegenüberliegenden Rinnenendbereiche einmünden. Insbesondere in Verbindung mit relativ großen langgestreckten Abzugsrinnen ist weiter eine Ausgestaltung vorteilhaft, bei der jeweils eine Abzugsleitung in die in Abzugsrinnenlängsrichtung gesehen gegenüberliegenden Rinnenendbereiche und zudem wenigstens eine Abzugsleitung in den zwischen diesen beiden Rinnenendbereichen liegenden Rinnenzwischenbereich einmünden. Je nach der Länge der Abzugsrinne können somit auch mehrere voneinander beabstandete Abzugsleitungen in den zwischen den beiden Rinnenendbereichen liegenden Rinnenzwischenbereich einmünden.

Um einen individuellen Abzug des Substrat-Sand-Gemisches aus den Abzugsrinnen sicherzustellen, ist gemäß einer besonders bevorzugten Ausgestaltung vorgesehen, dass zumindest die unterschiedlichen Abzugsrinnen zugeordneten Abzugsleitungen, bevorzugt jedoch jede der Abzugsleitungen, mittels eines von der Regel- und/oder Steuereinrichtung separat ansteuerbaren Absperrelementes öffenbar und absperrbar ist.

Für eine gezielte Zuführung des abgezogenen Substrat-Sand-Gemisches zum insbesondere durch einen Separator gebildeten Aufnahme- und/oder Absetzbehälter münden sämtliche Abzugsleitungen bevorzugt in eine, vorzugsweise mittels eines Absperrelementes gesteuert über die Steuer- und/oder Regeleinrichtung verschließbare und öffenbare, Sammelleitung.

Alternativ oder zusätzlich zu der eben beschriebenen, im Wesentlichen bodenseitigen Anordnung der Abzugsleitungen kann aber auch vorgesehen sein, dass die wenigstens eine Abzugsleitung oder wenigstens eine der Abzugsleitungen durch wenigstens eine, insbesondere gasdicht, durch eine Behälterwand (kann das Behälterdach und/oder die Behälterseitenwand und/oder die Behälterbodenwand sein) in den Behälterinnenraum eingeführte und im Behälterinnenraum bis in den Bereich einer zugeordneten Abzugsrinne geführte Abzugssonde gebildet ist. Besonders bevorzugt ist hier eine Ausführungsform, bei der die wenigstens eine Abzugsleitung oder wenigstens eine der Abzugsleitungen durch wenigstens eine, bezogen auf die Behälterhochachse, von oben durch das Behälterdach oder von der Seite durch die Behälterseitenwand gasdicht in den Behälterinnenraum eingeführte und im Behälterinnenraum bis in den Bereich einer zugeordneten Abzugsrinne geführte Abzugssonde gebildet ist.

Für eine besonders effektive Absaugung des in der zugeordneten Abzugsrinne angesammelten Substrat-Sand-Gemisches ist die wenigstens eine Abzugssonde bevorzugt höhenverstellbar gelagert, und zwar insbesondere dergestalt, dass eine behälterdachseitige angeordnete Schürze bei eingefülltem Flüssigsubstrat in dieses eintaucht und die Abzugssonde durch die Schürze hindurch gasdicht und höhenverstellbar in den Behälterinnenraum geführt ist.

Auch hier ist wieder eine Ausführungsform vorteilhaft, bei der die unterschiedlichen Abzugsrinnen zugeordneten Abzugssonden, bevorzugt sogar jede der Abzugssonden, mittels eines von der Regel- und/oder Steuereinrichtung separat ansteuerbaren Absperrelementes öffenbar und absperrbar ist.

Gemäß einer weiteren alternativen Ausgestaltung ist dem Flüssigsubstratbehälter wenigstens eine Strömungserzeugungseinrichtung, insbesondere wenigstens ein Rührwerk, zugeordnet oder weist der Flüssigsubstratbehälter wenigstens eine derartige Strömungserzeugungseinrichtung auf. Bei einer Betätigung der wenigstens einen Strömungserzeugungseinrichtung strömt ein im Flüssigsubstratbehälter aufgenommenes Flüssigsubstrat mit einer definiert vorgegebenen Substratströmungsrichtung und/oder mit einer definiert vorgegebenen Substratströmungsgeschwindigkeit oberhalb der und/oder entlang der Behälterbodenwand. Bevorzugt ist hierbei vorgesehen, dass die wenigstens eine Strömungserzeugungseinrichtung so im Flüssigsubstratbehälter angeordnet ist, dass das Flüssigsubstrat bei deren Betätigung in eine walzenförmige Strömung mit wenigstens einer sich, bezogen auf die Behälterhochachse, von oben nach unten, vorzugsweise um eine horizontale Behälterachse, bewegenden Strömungswalze, insbesondere mit behälterseitenwandseitiger Aufwärtsströmung, versetzbar ist. Eine derartige Substratströmung ist insbesondere dann vorteilhaft, wenn gemäß einer besonders bevorzugten Ausgestaltung vorgesehen ist, dass die Behälterbodenwand im Übergangsbereich zur Behälterseitenwand mehrere voneinander beabstandete, randseitige muldenartige Vertiefungen aufweist. Diese muldenartigen Vertiefungen sind dabei bevorzugt gleichmäßig voneinander beabstandet und/oder jeweils gleich ausgebildet.

Für eine besonders effektive Ansammlung und Anreicherung der Feststoffe als Substrat-Sand-Gemisch in den muldenartigen Vertiefungen ist bevorzugt vorgesehen, dass die muldenartigen Vertiefungen jeweils im Strömungsweg einer Strömungswalze des Flüssigsubstrates liegen. Besonders bevorzugt ist hierbei eine Ausgestaltung, bei der die muldenartigen Vertiefungen jeweils im Strömungsweg einer Strömungswalze mit behälterwandseitiger Aufwärtsströmung liegen, weil dadurch noch besser sichergestellt werden kann, dass die auf die Behälterwand auftreffenden Schweb- bzw. Feststoffe in Richtung Behälterbodenwand und damit in Richtung muldenartiger Vertiefung absinken.

Die muldenartigen Vertiefungen sind bevorzugt durch bodenwandseitige, randseitige Ausnehmungen gebildet, die nach Innen in Richtung zur Behältermitte hin eine rampenförmige Abschrägung aufweisen, die wiederum in einen in Richtung Behältermitte hin angrenzenden, vorzugsweise ebenen, Bodenwandbereich übergeht, und/oder die in Richtung zur Behälteraußenseite hin durch einen Behälterseitenwandbereich begrenzt sind. Die rampenförmige Abschrägung begünstigt das Ansammeln der Feststoffe in der jeweiligen muldenartigen Vertiefung. Der Behälterseitenwandbereich dient dagegen in einer Doppelfunktion gleichzeitig auch als die jeweilige muldenartige Vertiefung begrenzende Wand.

Bevorzugt mündet jeweils wenigstens eine, vorzugsweise eine einzige, Abzugsleitung in eine muldenartige Vertiefung, und zwar bevorzugt in einen, vorzugsweise ebenen, Muldenbodenbereich der jeweiligen muldenartigen Vertiefung. Mit einer derartigen Anordnung einer Abzugsleitung wird ein funktionssicheres Abziehen des sich in der jeweiligen muldenartigen Vertiefung ansammelnden Substrat-Sand-Gemisches möglich.

Für ein individuelles Abziehen der sich als Substrat-Sand-Gemisch in den jeweiligen muldenartigen Vertiefungen ansammelnden Feststoffe ist bevorzugt vorgesehen, dass zumindest die unterschiedlichen muldenartigen Vertiefungen zugeordneten Abzugsleitungen, bevorzugt jede der Abzugsleitungen, mittels eines von der Regel- und/oder Steuereinrichtung separat ansteuerbaren Absperrelementes öffenbar und absperrbar ist.

Des Weiteren ist es gemäß einer besonders bevorzugten konkreten Ausgestaltung vorteilhaft, wenn wenigstens ein Teil der Abzugsleitungen, vorzugsweise sämtliche Abzugsleitungen, in eine, vorzugsweise als Ringleitung ringförmig um den Substratbehälter umlaufenden, Sammelleitung münden, die zum Separator geführt ist und die, gesteuert über die Steuer- und/oder Regeleinrichtung, mittels eines Absperrelementes verschließbar und öffenbar ist. Damit wird eine kompakte und funktionssichere Zuführung des abgezogenen Substrat-Sand-Gemisches zum bevorzugt durch einen Separator gebildeten Aufnahme- und/oder Absetzbehälter möglich.

Das Absinken der Feststoffe und damit deren Ansammlung in den jeweiligen muldenartigen Vertiefungen als Substrat-Sand-Gemisch kann ferner zusätzlich noch dadurch begünstigt werden, dass der Bodenwandbereich in einem, zwischen den muldenförmigen Vertiefungen liegenden Ablenk-Bodenwandbereich wenigstens ein erhabenes Ablenkelement aufweist. Das wenigstens eine Ablenkelement kann sich dabei vom bodenwandseitigen Randbereich ausgehend nach Innen in Richtung zur Behältermitte hin verjüngen. Des Weiteren ist bevorzugt vorgesehen, dass das Ablenkelement dachförmig, insbesondere Sattel- oder Giebeldachförmig mit Firstkante, ausgebildet ist und zu den benachbarten muldenartigen Vertiefungen hin abfallende Seitenflächen aufweist. Ein derartiger Aufbau begünstigt das Absinken und Absetzen der Feststoffteilchen in einem erheblichen Maße.

Besonders bevorzugt ist hierbei eine Ausführungsform, bei der sich die, vorzugsweise gleich ausgebildeten, Ablenkelemente über die lediglich im randseitigen Bodenwandbereich ausgebildeten muldenartigen Vertiefungen hinaus nach innen in Richtung Behältermitte erstrecken. Dabei ist eine Ausführungsform bevorzugt, bei der der eine zylindrische Innenkontur aufweisende Flüssigsubstratbehälter eine senkrechte Behältermittelachse aufweist, auf die hin die inneren Enden der Ablenkelemente ausgerichtet sind.

Es versteht sich, dass die Ablenkelemente grundsätzlich auf unterschiedlichste Art und Weise ausgebildet sein können, so zum Beispiel durch separate Bauteile, wie zum Beispiel Blechbauteile, die mit der Behälterbodenwand fest verbunden, zum Beispiel fest verschraubt sind.

In Verbindung der eben beschriebenen Ausführungsform mit randseitig angeordneten muldenartigen Vertiefungen ist insbesondere ein Aufbau vorteilhaft, bei der die Strömungserzeugungseinrichtung durch ein mittig im Flüssigsubstratbehälter angeordnetes Zentralrührwerk gebildet ist, das sich in Behälterhochachsenrichtung erstreckt.

Beispielhafte Ausführungsformen eines erfindungsgemäßen Flüssigsubstratbehälters für eine Biogasanlage werden nachfolgend anhand einer Zeichnung näher erläutert.

Es zeigen:
- Fig. 1a: schematisch eine Draufsicht auf einen Flüssigsubstratbehälter einer Biogasanlage gemäß einer ersten Ausführungsform mit zwei sich diametral über eine Behälterbodenwand erstreckenden Abzugsrinnen,
- Fig. 1b: den Aufbau gemäß Fig. 1a mit zu den Abzugsrinnen geführten Abzugsleitungen,
- Fig. 1c: schematisch eine Schnittansicht entlang der Linie A-A der Fig. 1b,
- Fig. 1d: schematisch eine Schnittansicht entlang der Linie B-B der Fig. 1a,
- Fig. 2a: schematisch eine Draufsicht auf eine zweite Ausführungsform eines erfindungsgemäßen Flüssigsubstratbehälters mit einer einzigen diametral verlaufenden Abzugsrinne,
- Fig. 2b: schematisch eine Schnittansicht entlang der Linie C-C der Fig. 2a,
- Fig. 2c: schematisch eine Schnittansicht entlang der Linie D-D der Fig. 2a,
- Fig. 2d: schematisch eine der Fig. 2a entsprechende Darstellung mit im Detail dargestellter Abzugsrinne,
- Fig. 3a: eine schematische Draufsicht auf eine dritte Ausführungsform eines erfindungsgemäßen Flüssigsubstratbehälters mit einer von oben in den Substratbehälter einmündenden Abzugssonde,
- Fig. 3b: schematisch eine Querschnittsansicht des Aufbaus gemäß Fig. 3a,
- Fig. 4a: schematisch eine Seitenansicht eines Substratbehälters gemäß einer vierten Ausführungsform,
- Fig. 4b: schematisch eine Schnittansicht entlang der Linie E-E der Fig. 4a,
- Fig. 4c: schematisch eine Schnittansicht entlang der Linie F-F der Fig. 4a,
- Fig. 4d: schematisch eine Schnittansicht entlang der Linie G-G der Fig. 4e,
- Fig. 4e: schematisch eine Draufsicht auf die in Fig. 4d dargestellte Behälterbodenwand mit randseitigen muldenartigen Vertiefungen, und
- Fig. 5: schematisch eine beispielhafte Ausführungsform eines als Aufnahme- und/oder Absetzbehälter ausgebildeten Separators.

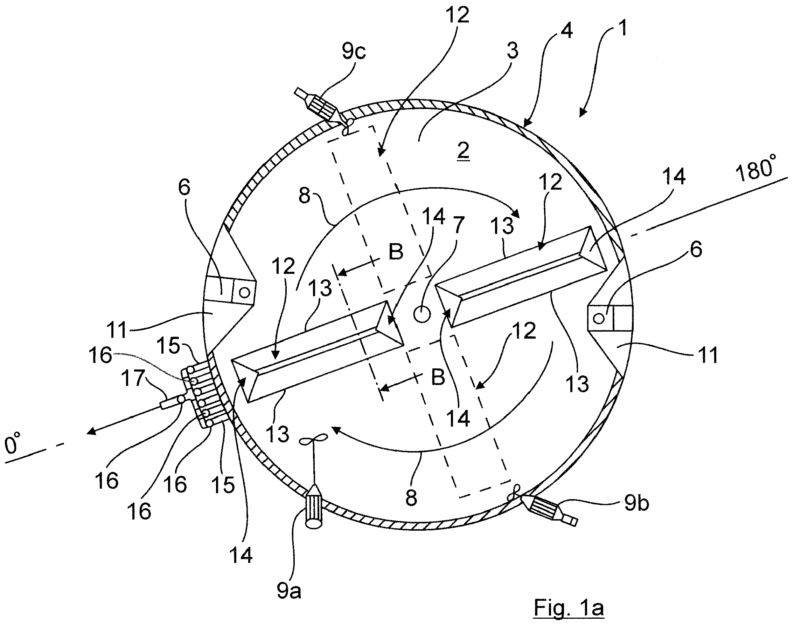

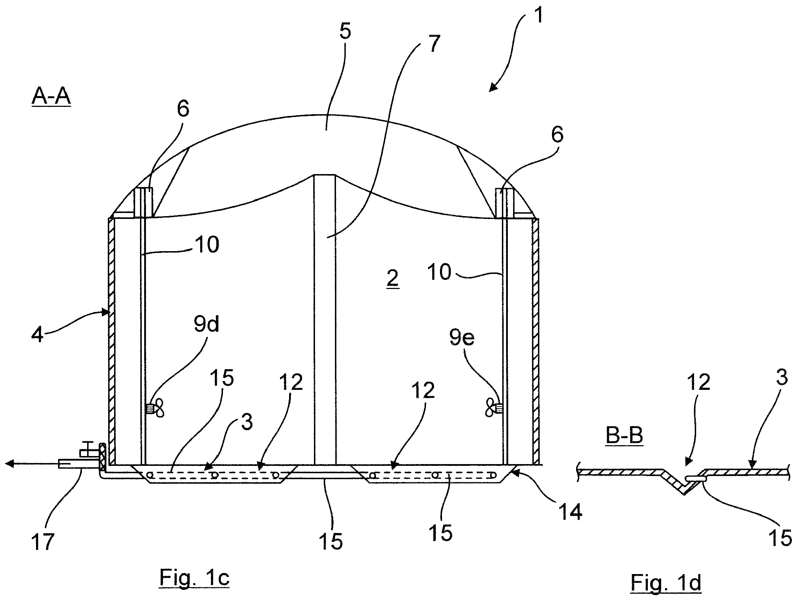

In der Fig. 1a ist schematisch und beispielhaft eine Draufsicht auf eine erste Ausführungsform eines erfindungsgemäßen Flüssigsubstratbehälters 1 für eine Biogasanlage gezeigt, der einen mit Flüssigsubstrat befüllbaren Behälterinnenraum 2, eine den Behälterinnenraum 2 bodenseitig begrenzende, hier eben ausgebildete Behälterbodenwand 3 sowie, was insbesondere aus der Fig. 1c ersichtlich ist, eine den Behälterinnenraum 2 umfangsseitig begrenzende Behälterseitenwand 4 aufweist. Die Abdeckung des Behälterinnenraums 2 erfolgt gemäß der Fig. 1c hier beispielhaft durch ein an sich bekanntes Foliendach 5.

Zur Erzeugung einer um die senkrechte Behältermittelachse 7 rotierenden Strömung (entsprechend der Pfeile 8) des hier nicht im Detail gezeigten Flüssigsubstrates sind umfangsseitig voneinander beabstandet mehrere Rührwerke 9a bis 9e vorgesehen, die hier lediglich beispielhaft und schematisch eingezeichnet sind. Während die Rührwerke 9a, 9b und 9c hier lediglich beispielhaft und schematisch beabstandet voneinander an der kreiszylindrischen Behälterseitenwand 4 angeordnet sind, sind die Rührwerke 9d und 9e (siehe Fig. 1c) höhenverstellbar und/oder verschwenkbar an einem senkrechten Führungsmast 10 gehaltert, wobei der Führungsmast 10 mit seinem oberen Ende in einem Serviceschacht 6 gelagert bzw. gehaltert ist, über den ein Zugang zu den Rührwerken 9d und 9e für Montage- und Serviceeinrichtungen möglich ist bzw. über die die Rührwerke 9d, 9e aus dem Behälterinnenraum 2 nach außen herausgehoben werden können. Die Serviceschächte 6 selbst sind, wie dies insbesondere aus der Fig. 1a ersichtlich ist, zum Beispiel in Verbindung mit einer Podestplatte 11 an der Behälterseitenwand 4 abgestützt und gehaltert.

Wie dies weiter aus der Zusammenschau der Fig. 1a bis 1d ersichtlich ist, sind in der Behälterbodenwand 3 hier beispielhaft zwei längliche bzw. langgestreckte und im Wesentlichen geradlinig verlaufende Abzugsrinnen ausgebildet, die sich jeweils von der Behältermittelachse 7 ausgehend nach außen zur Behälterseitenwand 4 erstrecken und somit eine im Wesentlichen diametral verlaufende Abzugsrinne zwischen diametral gegenüberliegenden Behälterbodenwandbereichen bzw. Behälterseitenwandbereichen des Flüssigsubstratbehälters 1 ausbilden.

Wie dies insbesondere der Fig. 1a weiter entnommen werden kann, sind die Abzugsrinnen hier bezüglich ihrer Längsseite im Wesentlichen quer zur rotierenden Substratströmungsrichtung im Behälterinnenraum 2 ausgerichtet und angeordnet, so dass ein um die senkrechte Behältermittelachse 7 rotierender Substratteil des Flüssigsubstrates die eine diametral verlaufende Abzugsrinne ausbildenden beiden Abzugsrinnen 12 während eines 360°-Umlaufs um die senkrechte Behältermittelachse 7 zweimal überstreicht, und zwar bei, bezogen auf die Abzugsrinnen 12, 0° bzw. 180°.

Wie in der Fig. 1a lediglich schematisch und strichliert dargestellt, könnten in der Behälterbodenwand 3 aber auch noch mehrere Abzugsrinnen 12 vorgesehen und ausgebildet sein, die sich dann insgesamt von der senkrechten Behältermittelachse 7 weg radial bzw. sternförmig nach außen erstrecken.

Wie dies aus der Fig. 1d, die einen Schnitt entlang der Linie B-B der Fig. 1a zeigt, ersichtlich ist, weisen die Abzugsrinnen einen bevorzugt V-förmigen oder trichterförmigen Querschnitt auf, so dass die Innenwände der Längsseiten 13 der Abzugsrinne 12 nach unten zur Bodenmitte hin schräg abfallen. Ebenso können, wie dies insbesondere aus der Fig. 1a ersichtlich ist, die gegenüberliegenden Breitseiten 14 schräg nach innen abfallende Innenwände aufweisen.

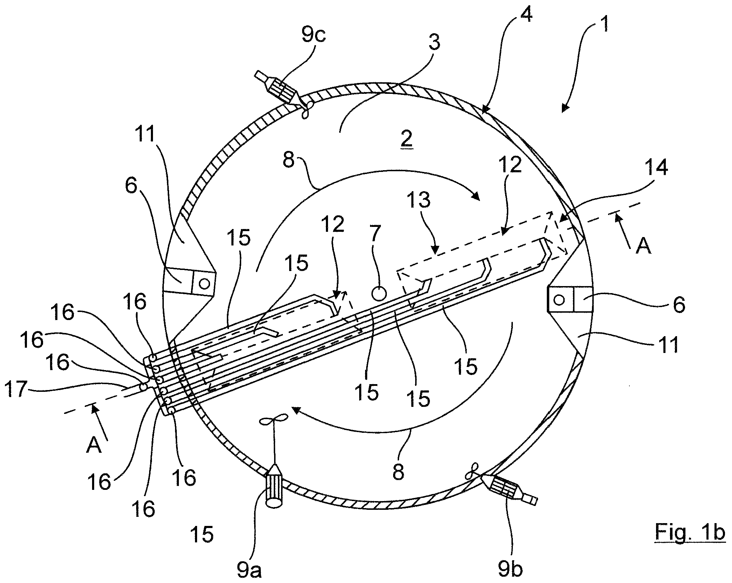

Wie dies insbesondere aus der Zusammenschau der Fig. 1b und 1c ersichtlich ist, münden in jede der Abzugsrinnen 12 hier mehrere, in Abzugsrinnenlängsrichtung voneinander beabstandete Abzugsleitungen 15 ein, die, bezogen auf die Behälterhochachse, in einer Horizontalebene verlaufen und hier jeweils in die schräg nach innen abfallenden Abzugsrinnen-Seitenwandbereiche einmünden. Wie dies insbesondere aus der Fig. 1b ersichtlich ist, mündet jeweils eine Abzugsleitung 15 in die in Abzugsrinnenlängsrichtung gesehen gegenüberliegenden Rinnenendbereiche ein, während eine Abzugsleitung 15 in den zwischen diesen Rinnenendbereichen liegenden Rinnenzwischenbereich einmündet.

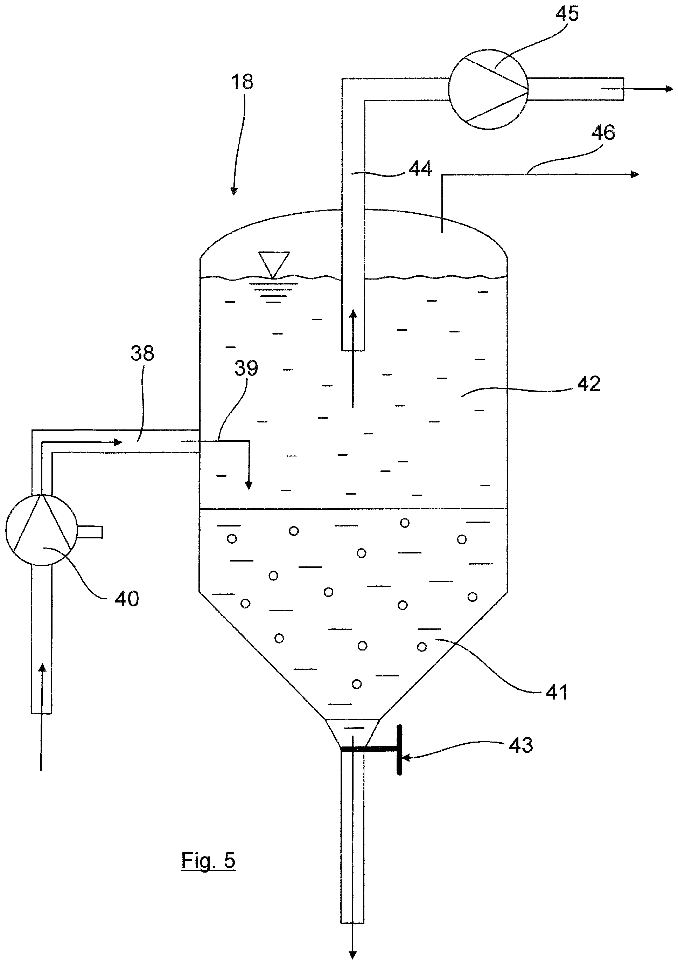

Wie dies weiter aus der Fig. 1b ersichtlich ist, ist hier beispielhaft jede der Abzugsleitungen 15 mittels eines von der Regel- und/oder Steuereinrichtung separat ansteuerbaren Absperrelementes 16, das zum Beispiel ein Schieber oder ein Ventil sein kann, öffenbar oder absperrbar, wobei hier zudem weiter vorgesehen ist, dass sämtliche Abzugsleitungen 16 in eine, hier ebenfalls mittels eines Absperrelementes 16 gesteuert über die Steuer- und/oder Regeleinrichtung verschließbare und öffenbare Sammelleitung 17 münden, die zu einem in der Fig. 5 gezeigten Separator 18 geführt ist.

Mit einem derartig aufgebauten Flüssigsubstratbehälter 1 kann das Flüssigsubstrat in die in der Fig. 1a und 1b schematisch gezeigte rotierende Strömung (Pfeile 8) versetzt werden, wodurch die einzelnen Substratteilbereiche die einzelnen Abzugsrinnen 12 im Verlauf mehrerer Umdrehungen mehrfach überstreichen. Die sich im Flüssigsubstrat befindlichen Feststoffe sinken dabei auf die Behälterbodenwand 3 ab und können sich dann, bedingt durch die rotierende Strömung und die Abzugsrinnen 12 als Substrat-Sand-Gemisch in den Abzugsrinnen 12 ablagern, von wo aus sie mittels der Abzugsleitungen 15 gesteuert mittels der Steuer- und/oder Regeleinrichtung abgezogen und über die Sammelleitung 17 dem Separator 18 zugeführt werden können. Die Absperrelemente 16 können dabei mittels der Steuer- und/oder Regeleinrichtung individuell angesteuert und somit eine individuelle Absaugung des Substrat-Sand-Gemisches aus den Abzugsrinnen 12 bewerkstelligt werden.

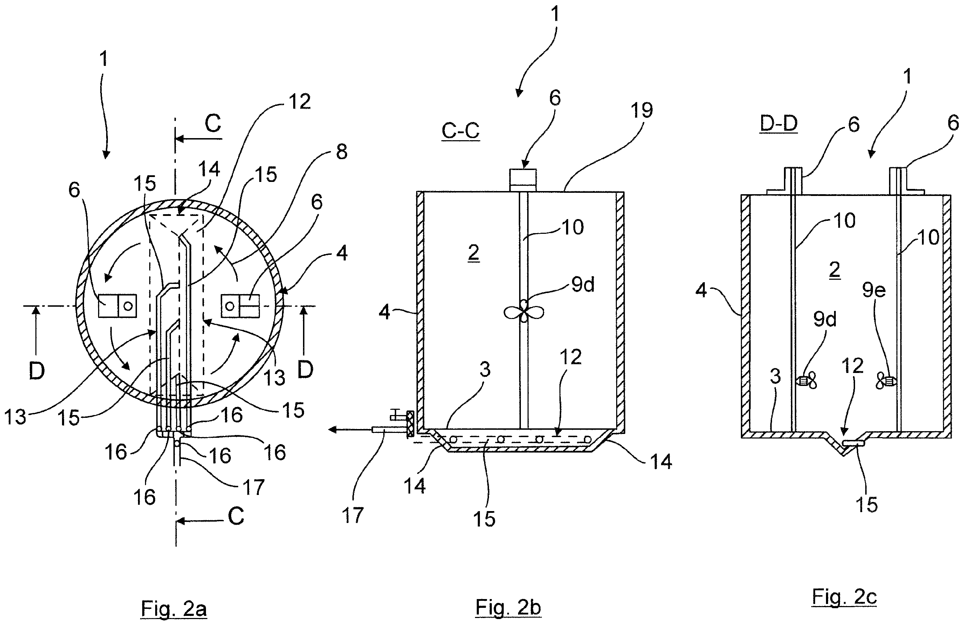

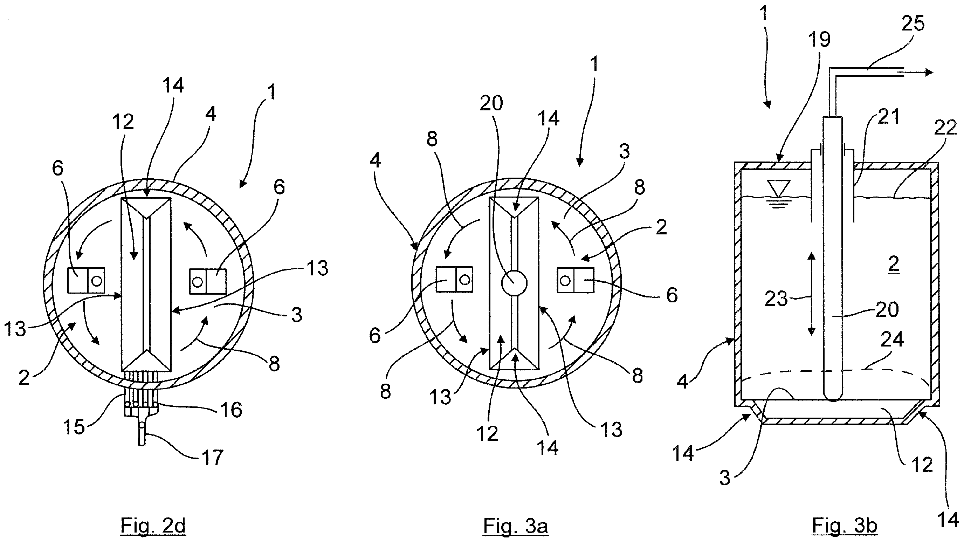

In den Fig. 2a bis 2d ist eine zur Ausgestaltung nach Fig. 1a bis 1d alternative Ausführungsform gezeigt, bei der der Flüssigsubstratbehälter 1 zum Beispiel einen geringeren Durchmesser aufweist als derjenige der Ausführungsform gemäß der Fig. 1d. Zudem erstreckt sich hier lediglich eine einzige Abzugsrinne 12 diametral zwischen gegenüberliegenden Randbereichen der Behälterbodenwand 3 bzw. zwischen gegenüberliegenden Seitenwandbereichen der Behälterseitenwand 4.

Auch hier münden in die Abzugsrinne 12, mit Bezug zur Behälterhochachsenrichtung, horizontal verlaufende Abzugsleitungen 15 beabstandet voneinander in die langgestreckte Abzugsrinne 12 ein, und zwar im Prinzip analog zu der Ausführungsform der Fig. 1a bis 1d, wobei zwischen den beiden in die gegenüberliegenden Rinnenendbereiche einmündenden Abzugsleitungen 15 hier, wiederum lediglich beispielhaft, zwei weitere voneinander beabstandete Abzugsleitungen 15 in den Rinnenzwischenbereich einmünden.

Im weiteren Unterschied zur Ausführungsform gemäß 1a bis 1d weist der Behälterinnenraum 2 hier kein kuppelartiges Foliendach, sondern einer ebene dachseitige Abdeckung 19 auf, an der hier lediglich äußerst schematisch und beispielhaft zwei Serviceschächte 6 mit zugeordneten Führungsmasten 10 und Rührwerken 9d und 9e angeordnet sind.

Ansonsten ist der Aufbau identisch mit derjenigen der Fig. 1a bis 1d, so dass bezüglich der betriebsmäßigen Absaugung des sich in der Abzugsrinne 12 ansammelnden Substrat-Sand-Gemisches in Verbindung mit einer rotierenden Strömung des Flüssigsubstrates auf die zuvor gemachten Ausführungen verwiesen wird.

In der Fig. 3a und 3b ist eine weitere alternative Ausführungsform eines erfindungsgemäßen Flüssigsubstratbehälters 1 gezeigt, der im Wesentlichen analog der Ausführungsform gemäß der Fig. 2a bis 2d ausgeführt ist, jedoch mit dem Unterschied, dass hier anstelle der horizontal verlaufenden, bodenseitigen Abzugsleitungen eine Abzugssonde 20 als Abzugsleitung vorgesehen ist, die, bezogen auf die Behälterhochachse hier lediglich beispielhaft von oben durch das Behälterdach bzw. die Dachwand 19 in den Behälterinnenraum 2 eingeführt und im Behälterinnenraum 2 bis in den Bereich der hier beispielhaft einzigen Abzugsrinne 12 geführt ist. Die hier beispielhaft lediglich eine einzige Abzugssonde 20 ist des weiteren bevorzugt höhenverstellbar gelagert, und zwar bevorzugt dergestalt, dass eine behälterdachseitig angeordnete Schürze 21 bei eingefülltem Flüssigsubstrat 22 in dieses eintaucht und die Abzugssonde 20 durch die Schürze 21 hindurch gasdicht und höhenverstellbar in den Behälterinnenraum geführt ist. Damit kann die Abzugssonde 20 entsprechend des Pfeils 23 in der Fig. 3b in Hochachsenrichtung verlagert und verstellt werden, was insbesondere dann von Vorteil ist, wenn sich in einem, insbesondere relativ kleinvolumigen, Flüssigsubstratbehälter 1 vor allem auch im Bereich oberhalb der Abzugsrinne 12 eine relativ große Menge an abzusaugendem Substrat-Sand-Gemisch 24 ansammelt, wie dies in der Fig. 3b lediglich äußerst schematisch dargestellt ist.

Auch hier kann die Abzugssonde wieder mittels der Regel- und/oder Steuereinrichtung angesteuert werden, um das Absaugen des Substrat-Sand-Gemisches aus der Abzugsrinne 12 zu steuern. Von der Abzugssonde 20 führt eine entsprechend Rückführleitung 25 dann zum Separator 18, wie er beispielhaft in der Fig. 5 dargestellt ist.

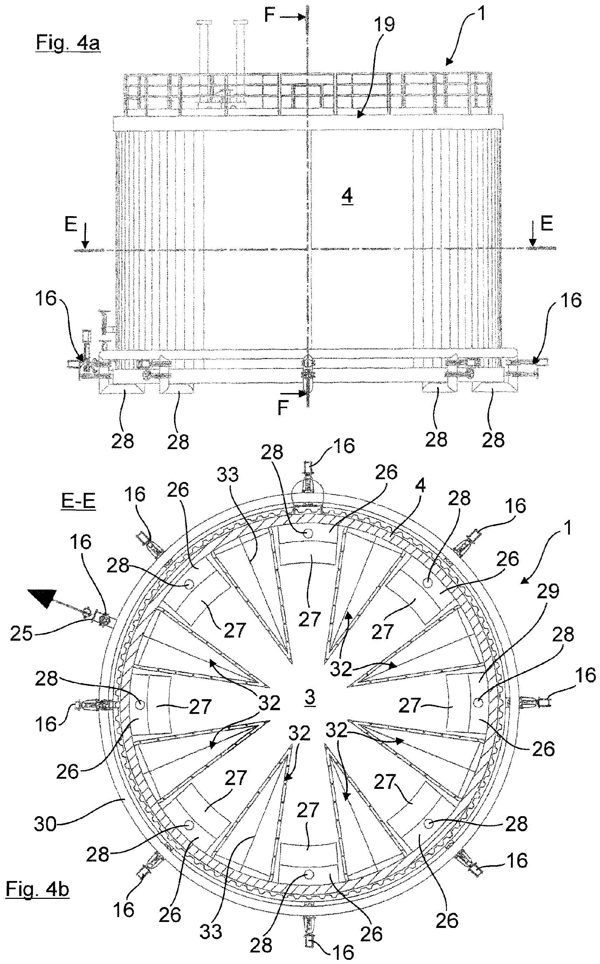

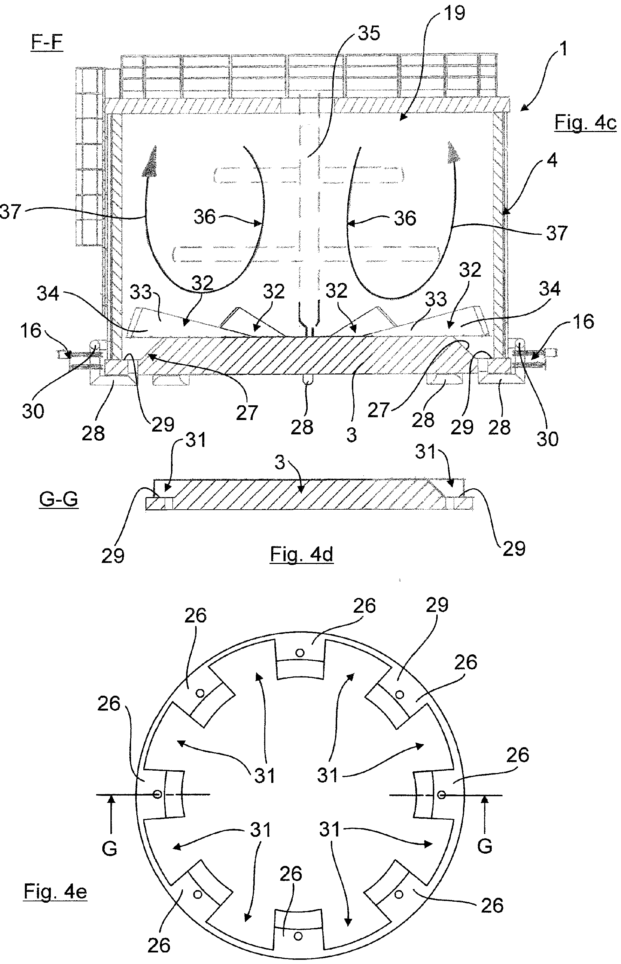

In den Fig. 4a bis 4d ist schließlich eine weitere alternative, vierte Ausführungsform eines erfindungsgemäßen Flüssigsubstratbehälters 1 gezeigt, bei dem die Behälterbodenwand 3 im Übergangsbereich zur Behälterseitenwand 4 mehrere voneinander beabstandete, randseitige muldenartige Vertiefungen 26 aufweist, die hier beispielhaft und bevorzugt gleichmäßig voneinander beabstandet sowie im Wesentlichen gleich ausgebildet sind.

Die muldenartigen Vertiefungen 26 sind hier jeweils durch bodenwandseitige, randseitige Ausnehmungen gebildet, die nach Innen zur Behältermitte hin eine rampenförmige Abschrägung 27 aufweisen, die wiederum in einen in Richtung Behältermitte hin angrenzenden, hier beispielhaft ebenen Bodenwandbereich übergeht. Des Weiteren sind die muldenartigen Vertiefungen 26 zur Behälteraußenseite durch einen Behälterseitenwandbereich begrenzt.

Den muldenartigen Vertiefungen 26 ist jeweils eine einzige Abzugsleitung 28 zugeordnet, die in einem ebenen Muldenbodenbereich 29 der jeweiligen muldenartigen Vertiefung 26 mündet.

Die einzelnen Abzugsleitungen 28 sind auch hier jeweils mittels eines von der Regel- und/oder Steuereinrichtung separat ansteuerbaren Absperrelementes öffenbar und absperrbar.

Zudem münden hier sämtliche Abzugsleitungen 28 in einer als Ringleitung ringförmig um den Flüssigsubstratbehälter 1 umlaufenden Sammelleitung 30, die zum Separator 18 geführt ist, und die, gesteuert über die Steuer- und/oder Regeleinrichtung, mittels eines Absperrelementes verschließbar und öffenbar ist.

Die Behälterbodenwand 3 weist hier zudem in einem zwischen den muldenförmigen Vertiefungen 26 liegenden Ablenk-Bodenwandbereich 31 ein erhabenes Ablenkelement 32 auf, das sich vom bodenwandseitigen Randbereich ausgehend nach Innen in Richtung zur Mitte hin verjüngt und hier zudem sattel- bzw. giebeldachförmig mit Firstkante 33 ausgebildet ist und jeweils zu den benachbarten muldenartigen Vertiefungen 26 hin abfallende Seitenflächen 34 aufweist.

Wie dies weiter aus den Fig. 4a bis 4d ersichtlich ist, erstrecken sich die, hier vorzugsweise gleich ausgebildeten Ablenkelemente 32 jeweils über die lediglich im randseitigen Bodenwandbereich ausgebildeten muldenartigen Vertiefungen 26 hinaus nach Innen in Richtung zur Behältermitte, wobei der eine zylindrische Innenkontur aufweisende Flüssigsubstratbehälter 1, der hier lediglich beispielhaft ein horizontales Behälterdach 19 aufweist, eine durch ein Zentralrührwerk 35 gebildete senkrechte Behältermittelachse aufweist, auf die hin die inneren Enden der Ablenkelemente 32 ausgerichtet sind.

Die Ablenkelemente 32 können zum Beispiel aus einem Blech hergestellt sein sowie zum Beispiel als separate Bauteile fest mit der Behälterbodenwand verbunden, insbesondere verschraubt sein.

Das Zentralrührwerk 35 ist bevorzugt so ausgebildet, dass das Flüssigsubstrat bei dessen Betätigung in eine walzenförmige Strömung mit wenigstens einer sich, bezogen auf die Behälterhochachse, von oben nach unten, um eine horizontale Behälterachse bewegenden Strömungswalze 36 (siehe Fig. 4c) mit behälterseitenwandseitiger Aufwärtsströmung 37 versetzbar ist.

Mit einem derartigen Aufbau wird sichergestellt, dass die im Flüssigsubstrat enthaltenden Feststoffe sich bevorzugt randseitig anreichern und dann nach unten absinken, wobei die Ablenkelemente 32 vorteilhaft bewirken, dass die Sedimente in Richtung zu den muldenartigen Vertiefungen 26 hin abgelenkt bzw. geleitet werden, von wo sie dann mittels der Abzugsleitungen 28 als Substrat-Sand-Gemisch abgezogen und dem Separator 18 zugeführt werden können.

In der Fig. 5 ist schließlich eine beispielhafte schematische Prinzipskizze eines Separators 18 gezeigt, dem über die Zuführleitung 38, die zum Beispiel mit der Sammelleitung 17, der Rückführleitung 25 oder der Sammelleitung 30 der vorhin beschriebenen unterschiedlichen Ausführungsformen strömungsverbunden sein kann, um das aus dem Flüssigsubstratbehälter 1 mittels der jeweiligen Abzugsleitungen 15 bzw. der ebenfalls eine Abzugsleitung ausbildenden Abzugssonde 20 abgezogene Substrat-Sand-Gemisch in den Innenraum des Separators 18 zu führen (Pfeil 39). Die Abzugsleitungen sind dabei jeweils Bestandteil einer vorzugsweise als Vakuum- und/oder Unterdruck-Absaugeinrichtung ausgebildeten Absaugeinrichtung, die eine Vakuum- und/oder Unterdruckpumpe 40 aufweist, mittels der das pumpbare Substrat über die jeweils geöffneten Abzugsleitungen aus der jeweiligen Abzugsrinne 12 bzw. der jeweiligen muldenartigen Vertiefung 26 absaugbar ist.

Der Separator 18 kann zudem selbst auch als Vakuum- und/oder Unterdruckbehälter ausgebildet sein, in dem das Substrat-Sand-Gemisch mit einem gegenüber dem atmosphärischen Druck geringeren Druck aufgenommen ist. Im Separator 18 wird das Substrat-Sand-Gemisch in eine Sandphase 41 und eine Substratphase 42 getrennt, wobei sich die Sandphase 41 dann im Separator 18 bodenseitig ansammelt und dort zum Beispiel gezielt gesteuert durch Öffnen eines Absperrelementes 43, das von der Steuer- und/oder Regeleinrichtung angesteuert wird, abgezogen werden kann. Ebenso kann die Substratphase 42 als Flüssigphase über eine Rückführleitung 44 in den Flüssigsubstratbehälter 1 rückgeführt werden, was beispielsweise mit Hilfe einer Pumpe 45 als Fördereinrichtung erfolgen kann. Falls es im Separator 18 zu einer Nachvergärung kommen sollte und sich Gas bildet, kann dies ebenfalls mittels einer Abzugsleitung 46 abgezogen und einer weiteren Verwendung zugeführt werden.

### Bezugszeichenliste

| | | | |
|---|---|---|---|
| 1 | Flüssigsubstratbehälter | 30 | Sammelleitung |
| 2 | Behälterinnenraum | 31 | Ablenk-Bodenwandbereich |
| 3 | Behälterbodenwand | 32 | Ablenkelement |
| 4 | Behälterseitenwand | 33 | Firstkante |
| 5 | Foliendach | 34 | abfallende Seitenflächen |
| 6 | Serviceschächte | 35 | Zentralrührwerk |
| 7 | Behältermittelachse | 36 | Strömungswalze |
| 8 | Pfeile | 37 | Aufwärtsströmung |
| 9a bis 9e | Rührwerke | 38 | Zuführleitung |
| 10 | Führungsmast | 39 | Pfeil |
| 11 | Podestplatte | 40 | Vakuum- und/oder Unterdruckpumpe |
| 12 | Abzugsrinne | | |
| 13 | Längsseiten | 41 | Sandphase |
| 14 | Breitseiten | 42 | Substratphase |
| 15 | Abzugsleitungen | 43 | Absperrelement |
| 16 | Absperrelement | 44 | Rückführleitung |
| 17 | Sammelleitung | 45 | Pumpe |
| 18 | Separator | 46 | Gasabzug |
| 19 | Dachwand | | |
| 20 | Abzugsonde | | |
| 21 | Schürze | | |
| 22 | Flüssigsubstrat | | |
| 23 | Pfeil | | |
| 24 | Substrat-Sand-Gemisch | | |
| 25 | Rückführleitung | | |
| 26 | muldenartige Vertiefungen | | |
| 27 | Abschrägung | | |
| 28 | Abzugsleitung | | |
| 29 | ebener Muldenbodenbereich | | |

## Patentansprüche

1. Flüssigsubstratbehälter (1) für eine Biogasanlage, mit einem Behälterinnenraum (2), der mit einem Flüssigsubstrat (22) befüllbar ist und mit einer den Behälterinnenraum (2) bodenseitig begrenzenden, insbesondere wenigstens bereichsweise ebenen, Behälterbodenwand (3),
**dadurch gekennzeichnet,**
**dass** in der Behälterbodenwand (3) wenigstens eine sich über einen vorgegebenen Teilbereich der Behälterbodenwand (3) erstreckende, muldenartige Vertiefung (12; 26) ausgebildet ist, zu der und/oder in die wenigstens eine Abzugsleitung (15) einer Abzugseinrichtung geführt ist,
**dass** die Abzugseinrichtung eine Steuer- und/oder Regeleinrichtung aufweist, mittels der die Abzugseinrichtung dergestalt betätigbar ist, dass ein sich in der wenigstens einen muldenartigen Vertiefung (12; 26) betriebsmäßig ansammelndes Substrat-Sand-Gemisch (24) über die wenigstens eine Abzugsleitung (15) aus der wenigstens einen muldenartigen Vertiefung (12; 26) und damit aus dem Behälterinnenraum (2) abziehbar ist, und
**dass** die Abzugseinrichtung ferner wenigstens einen mit der wenigstens einen Abzugsleitung (15) strömungsverbundenen Aufnahme- und/oder Absetzbehälter (18), insbesondere einen Separator, aufweist, in dem das über die wenigstens eine Abzugsleitung (15) abgezogene Substrat-Sand-Gemisch (24) aufnehmbar ist, insbesondere in eine Substratphase (42) und in eine Sandphase (41) trennbar ist.

2. Flüssigsubstratbehälter nach Anspruch 1, **dadurch gekennzeichnet, dass** die wenigstens eine Abzugsleitung (15) eine Absaugleitung einer Absaugeinrichtung als Abzugseinrichtung ist, wobei bevorzugt vorgesehen ist, dass die Absaugeinrichtung eine Vakuum-und/oder Unterdruck-Absaugeinrichtung ist, die eine Vakuum- und/oder Unterdruckpumpe (40) aufweist, mittels der das pumpbare Substrat-Sand-Gemisch über die wenigstens eine Absaugleitung (15) aus der wenigstens einen muldenartigen Vertiefung (12; 26) absaugbar ist, und/oder die einen Vakuum- und/oder Unterdruckbehälter als Separator aufweist, in dem das Substrat-Sand-Gemisch mit einem gegenüber dem atmosphärischen Druck geringeren Druck aufgenommen ist.

3. Flüssigsubstratbehälter nach Anspruch 1 oder 2, **dadurch gekennzeichnet, dass** dem Flüssigsubstratbehälter (1) wenigstens eine Strömungserzeugungseinrichtung, insbesondere wenigstens ein Rührwerk, zugeordnet ist oder aufweist, bei deren Betätigung ein im Flüssigsubstratbehälter (1) aufgenommenes Flüssigsubstrat (22) mit einer definiert vorgegebenen Substratströmungsrichtung und/oder mit einer definiert vorgegebenen Substratströmungsgeschwindigkeit oberhalb der und/oder entlang der Behälterbodenwand (3) strömt, wobei bevorzugt vorgesehen ist, dass die wenigstens eine Strömungserzeugungseinrichtung so im Flüssigsubstratbehälter (1) angeordnet ist, dass das Flüssigsubstrat bei deren Betätigung in eine walzenförmige Strömung mit wenigstens einer sich, bezogen auf die Behälterhochachse, von oben nach unten, vorzugsweise um eine horizontale Behälterachse, bewegender Strömungswalze (36), insbesondere mit behälterseitenwandseitiger Aufwärtsströmung, versetzbar ist.

4. Flüssigsubstratbehälter nach einem der vorhergehenden Ansprüche, **dadurch gekennzeichnet, dass** die Behälterbodenwand (3) im Übergangsbereich zur Behälterseitenwand (4) mehrere voneinander beabstandete, randseitige muldenartige Vertiefungen (26) aufweist, wobei bevorzugt vorgesehen ist, dass die muldenartigen Vertiefungen (26) gleichmäßig voneinander beabstandet und/oder gleich ausgebildet sind.

5. Flüssigsubstratbehälter nach Anspruch 3 und 4, **dadurch gekennzeichnet, dass** die muldenartigen Vertiefungen (26) jeweils im Strömungsweg einer Strömungswalze (36) des Flüssigsubstrates, insbesondere einer Strömungswalze (36) mit behälterwandseitiger Aufwärtsströmung (37), liegen.

6. Flüssigsubstratbehälter nach Anspruch 4 oder 5, **dadurch gekennzeichnet, dass** die muldenartigen Vertiefungen (26) durch bodenwandseitige, randseitige Ausnehmungen gebildet sind, die nach innen in Richtung zur Behältermitte hin eine rampenförmige Abschrägung (27) aufweisen, die wiederum in einen in Richtung Behältermitte hin angrenzenden, vorzugsweise ebenen, Bodenwandbereich übergeht, und/oder die in Richtung zur Behälteraußenseite hin durch einen Behälterseitenwandbereich begrenzt sind.

7. Flüssigsubstratbehälter nach einem der Ansprüche 4 bis 6, **dadurch gekennzeichnet, dass** jeweils wenigstens eine, vorzugsweise eine einzige, Abzugsleitung (15) in eine muldenartige Vertiefung (26) mündet, insbesondere in einem, vorzugsweise ebenen, Muldenbodenbereich (29) der jeweiligen muldenartigen Vertiefung (26) mündet.

8. Flüssigsubstratbehälter nach einem der Ansprüche 4 bis 7, **dadurch gekennzeichnet, dass** zumindest die unterschiedlichen muldenartigen Vertiefungen (26) zugeordneten Abzugsleitungen (15), bevorzugt jede der Abzugsleitungen, mittels eines von der Regel- und/oder Steuereinrichtung separat ansteuerbaren Absperrelementes (16) öffenbar und absperrbar ist.

9. Flüssigsubstratbehälter nach Anspruch 7 oder 8, **dadurch gekennzeichnet, dass** wenigstens ein Teil der Abzugsleitungen (15), vorzugsweise sämtliche Abzugsleitungen (15), in einer, vorzugsweise als Ringleitung ringförmig um den Flüssigsubstratbehälter (1) umlaufenden, Sammelleitung (30) münden, die zum Aufnahme- und/oder Absetzbehälter (18), insbesondere zu einem Separator, geführt ist und die, gesteuert über die Steuer- und/oder Regeleinrichtung, mittels eines Absperrelementes (16) verschließbar und öffenbar ist.

10. Flüssigsubstratbehälter nach einem der Ansprüche 7 bis 9, **dadurch gekennzeichnet, dass** die Behälterbodenwand (3) in einem zwischen den muldenförmigen Vertiefungen (26) liegenden Ablenk-Bodenwandbereich (31) wenigstens ein erhabenes Ablenkelement (32) aufweist.

11. Flüssigsubstratbehälter nach Anspruch 10, **dadurch gekennzeichnet, dass** sich das wenigstens eine Ablenkelement (32) vom bodenwandseitigen Randbereich ausgehend nach innen in Richtung zur Behältermitte hin verjüngt und/oder dass das wenigstens eine Ablenkelement (32) dachförmig, insbesondere sattel- oder giebeldachförmig mit Firstkante (33), ausgebildet ist und zu den benachbarten muldenartigen Vertiefungen (26) hin abfallende Seitenflächen (34) aufweist.

12. Flüssigsubstratbehälter nach Anspruch 10 oder 11, **dadurch gekennzeichnet, dass** sich die, vorzugsweise gleich ausgebildeten, Ablenkelemente (32) über die lediglich im randseitigen Bodenwandbereich ausgebildeten muldenartigen Vertiefungen (26) hinaus nach innen in Richtung Behältermitte erstrecken, insbesondere der eine zylindrische Innenkontur aufweisende Flüssigsubstratbehälter (1) eine senkrechte Behältermittelachse aufweist, auf die hin die inneren Enden der Ablenkelemente (32) ausgerichtet sind.

13. Flüssigsubstratbehälter nach einem der Ansprüche 3 bis 12, **dadurch gekennzeichnet, dass** die Strömungserzeugungseinrichtung ein mittig im Flüssigsubstratbehälter angeordnetes Zentralrührwerk (35) ist.

14. Flüssigsubstratbehälter nach einem der vorhergehenden Ansprüche, **dadurch gekennzeichnet, dass** vom als Separator ausgebildeten Aufnahme- und/oder Absetzbehälter (18) wenigstens eine Rückführleitung (44) zum Flüssigsubstratbehälter (1) geführt ist.
